(19) Europäisches Patentamt
European Patent Office
Office européen des brevets

(11) Numéro de publication : **0 134 179**
**B1**

(12) # FASCICULE DE BREVET EUROPÉEN

(45) Date de publication du fascicule du brevet : **28.09.88**

(51) Int. Cl.⁴ : **C 07 C 69/738**, C 07 C 69/035, C 07 C 67/08, A 61 K 31/215

(21) Numéro de dépôt : **84401718.6**

(22) Date de dépôt : **24.08.84**

(54) **Nouveaux dérivés de l'acide 4-phényl 4-oxo-buten 2-oïque, leur procédé de préparation, des médicaments et des compositions les renfermant.**

(30) Priorité : **25.08.83 IT 2264883**

(43) Date de publication de la demande : **13.03.85 Bulletin 85/11**

(45) Mention de la délivrance du brevet : **28.09.88 Bulletin 88/39**

(84) Etats contractants désignés : **AT BE CH DE FR GB IT LI LU NL SE**

(56) Documents cités :
**DE-A- 3 228 842**
**FR-A- 2 515 038**
**Le dossier contient des informations techniques présentées postérieurement au dépôt de la demande et ne figurant pas dans le présent fascicule.**

(73) Titulaire : **ROUSSEL-UCLAF**
**35, boulevard des Invalides**
**F-75007 Paris (FR)**

(72) Inventeur : **Bianchi, Mario**
**Via G. Giusti, 3**
**I-20048 Carate Brianza (Milan) (IT)**
Inventeur : **Barzaghi, Fernando**
**Via Monteverdi, 21**
**I-20052 Monza (Milan) (IT)**

(74) Mandataire : **Fritel, Hubert et al**
**Département des Brevets ROUSSEL UCLAF B.P. no 9**
**F-93230 Romainville (FR)**

**EP 0 134 179 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

L'invention a pour objet de nouveaux dérivés de l'acide 4-phényl 4-oxo buten 2-oïque, leur procédé de préparation, leur application comme médicaments et les compositions les renfermant.

On connaissait des dérivés de l'acide 4-phényl-4-oxobuten-2-oïque (cf. FR-A-2 515 038), on vient de découvrir de nouveaux dérivés de cet acide doués de propriétés antisécrétoires gastriques inattendues.

L'invention a pour objet les composés de formule (I) :

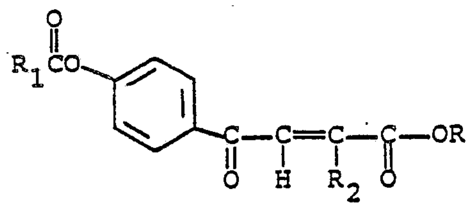

(I)

dans laquelle $R_1$ représente un radical alkyle renfermant de 1 à 18 atomes de carbone, $R_2$ représente un atome d'hydrogène ou un radical alkyle renfermant de 1 à 8 atomes de carbone et R représente un atome d'hydrogène ou un radical alkyle renfermant de 1 à 8 atomes de carbone, ainsi que les sels alcalins, alcalino-terreux ou d'amines des produits de formule (I) dans lesquels R représente un atome d'hydrogène.

Parmi les valeurs préférées de $R_1$, on peut citer les radicaux alkyle renfermant de 1 à 6 atomes de carbone et notamment les radicaux méthyle, éthyle, n-propyle et n-butyle.

Lorsque $R_2$ représente un radical alkyle, il s'agit de préférence du radical méthyle.

Lorsque R représente un radical alkyle, il s'agit de préférence d'un radical renfermant de 1 à 5 atomes de carbone, par exemple le radical méthyle, éthyle, n-propyle, isopropyle, n-butyle, isobutyle, terbutyle ou n-pentyle.

Les sels alcalins ou alcalino-terreux des produits de formule (I) dans laquelle R représente un atome d'hydrogène sont de préférence les sels de sodium, de potassium, de lithium ou de calcium.

Les sels d'amines des produits de formule (I) dans laquelle R représente un atome d'hydrogène, sont les sels d'amines usuelles. Parmi les amines usuelles, on peut citer les monoalcoylamines, telles que par exemple, la méthylamine, l'éthylamine, la propylamine, les dialcoylamines, telles que par exemple, la diméthylamine, la diéthylamine, la di-n-propylamine, les trialcoylamines, telles que la triéthylamine. On peut citer également la pipéridine, la morpholine, la pipérazine ou la pyrrolidine.

Les produits de formule (I) peuvent exister sous forme d'isomère E ou Z (trans ou cis).

L'invention a notamment pour objet les composés de formule (I) pour lesquels R représente un atome d'hydrogène ainsi que leurs sels alcalins, alcalino-terreux ou d'amines et notamment ceux pour lesquels $R_2$ représente un atome d'hydrogène.

L'invention a tout particulièrement pour objet les composés de formule (I) pour lesquels $R_1$ représente un radical méthyle ou un radical éthyle.

L'invention a tout spécialement pour objet les produits dont la préparation est donnée plus loin dans la partie expérimentale ainsi que leurs sels alcalins, alcalino-terreux ou d'amines.

Les produits de formule (I) ci-dessus sous les différentes formes isomères possibles, ainsi que les sels alcalins, alcalino-terreux ou d'amines desdits produits de formule (I) dans laquelle R représente un atome d'hydrogène, présentent d'intéressantes propriétés pharmacologiques ; ils manifestent notamment une importante activité antiulcéreuse. De plus, mis en contact avec la muqueuse gastrique, ils manifestent une activité antisécrétoire gastrique et cytoprotectrice.

Ces propriétés justifient leur application en thérapeutique et l'invention a également pour objet, à titre de médicaments, les produits de formule (I) tels que définis ci-dessus, sous les différentes formes isomères possibles, ainsi que les sels alcalins, alcalino-terreux ou d'amines pharmaceutiquement acceptables desdits produits de formule (I) dans laquelle R représente un atome d'hydrogène.

L'invention a tout particulièrement pour objet, à titre de médicaments, les composés de formule (I), décrits en exemple et leurs sels alcalins, alcalino-terreux et d'amines, pharmaceutiquement acceptables.

L'ensemble des produits de formule (I) constituent selon l'invention, des médicaments très utiles en thérapeutique humaine, notamment pour le traitement des hyperchlorhydries, des ulcères gastriques et gastriduodénaux des gastrites, des hernies hiatales, des affections gastriques et gastroduodénales s'accompagnant d'hyperacidité gastrique.

La posologie, variable selon le produit utilisé et l'affection en cause, peut s'échelonner par exemple entre 0,05 g et 2 g par jour chez l'adulte par voie orale.

La présente demande a encore pour objet les compositions pharmaceutiques qui contiennent, à titre de principe actif, au moins un des médicaments précités. Ces compositions sont réalisées de façon à pouvoir être administrées par la voie digestive ou parentérale.

Elles peuvent être solides ou liquides et se présenter sous les formes pharmaceutiques couramment utilisées en médecine humaine, comme par exemple, les comprimés simples ou dragéifiés, les gélules, les

granulés, les suppositoires, les préparations injectables ; elles sont préparées selon les méthodes usuelles.

Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet un procédé de préparation des composés de formule (I), ainsi que de leurs sels, caractérisé en ce que l'on soumet un composé de formule (II) :

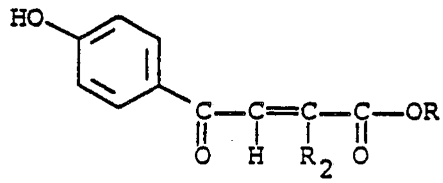

(II)

dans laquelle R et $R_2$ sont définis comme précédemment, à l'action d'un acide $R_1CO_2H$ ou d'un dérivé fonctionnel de cet acide pour obtenir le composé de formule (I) correspondant que, le cas échéant, l'on salifie ou estérifie.

Comme dérivé fonctionnel d'acide, on utilise de préférence un halogénure d'acide, par exemple un chlorure d'acide ou un anhydride.

Les sels alcalins, alcalino-terreux ou d'amines de produits de formule (I) peuvent être préparés par un procédé usuel tel que par exemple, par action sur lesdits produits de formule (I) des bases correspondantes ou par réaction de double décomposition ou par tous procédés usuels connus pour ce type d'acides carboxyliques α, β éthyléniques.

La réaction de salification est réalisée, de préférence, dans un solvant ou un mélange de solvants, tel que l'eau, l'éther éthylique, l'acétone, l'acétate d'éthyle, le tétrahydrofuranne ou le dioxanne.

Les produits de formule (I) dans laquelle R représente un radical alkyle contenant de 1 à 8 atomes de carbone peuvent être préparés soit en utilisant au départ des composés de formule (II) dans laquelle R représente un radical alkyle, soit à partir des produits de formule (I) dans laquelle R représente un atome d'hydrogène, de façon usuelle, par action d'un alcool de formule ROH, de préférence en milieu acide. L'acide peut être par exemple l'acide chlorhydrique, l'acide phosphorique ou l'acide paratoluène sulfonique.

Les composés de formule (II) utilisés comme produits de départ sont des produits connus qui peuvent être préparés, par exemple, selon la méthode décrite dans la demande de brevet anglais 2 108 385.

Exemple 1 : Acide (E) 4-(4-acétoxyphényl) 4-oxo buten-2-oïque

On chauffe pendant 1 heure et demie à 50 °C, une solution de 6 g d'acide (E) 4-(4-hydroxyphényl) 4-oxo buten-2-oïque (décrit dans J.A.C.S. 70, 3 356, 1948) dans 50 $cm^3$ d'anhydride acétique. On refroidit à température ambiante, dilue à l'eau, concentre puis extrait à l'acétate d'éthyle. On sépare la phase organique, la sèche et évapore le solvant. On recristallise le résidu dans l'éthanol à 70 % et obtient 4,5 g de produit attendu. F = 154-6 °C.

Analyse : $C_{12}H_{10}O_5$

| | | |
|---|---|---|
| Calculé : | C% 61,54 | H% 4,30 |
| Trouvé : | 61,48 | 4,18 |

Exemple 2 : Acide (E) 4-(4-propanoyloxyphényl) 4-oxo buten-2-oïque

On chauffe à 100 °C pendant 2 heures un mélange de 2,5 g d'acide (E) 4-(4-hydroxyphényl) 4-oxo buten-2-oïque et 25 $cm^3$ d'anhydride propanoïque. On évapore l'excès d'anhydride, reprend au benzène et évapore à sec. On chromatographie le résidu sur silice en éluant au mélange benzène-acétate d'éthyle-acide acétique (50-50-1). On cristallise le produit dans l'eau et obtient après séchage 1,9 g de produit attendu. F = 140-2 °C.

Analyse : $C_{13}H_{12}O_5$

| | | |
|---|---|---|
| Calculé : | C% 62,90 | H% 4,87 |
| Trouvé : | 62,96 | 4,77 |

Formes pharmaceutiques.

Exemple 3 : Comprimés

On a préparé des comprimés répondant à la formule suivante :

— Produit de l'exemple 1 100 mg
— Excipient q.s. pour un comprimé terminé à 300 mg
(Détail de l'excipient : lactose, amidon de blé, amidon traité, amidon de riz, stéarate de magnésium, talc).

Exemple 4 : Gélules

On a préparé des gélules répondant à la formule suivante :
— Produit de l'exemple 2 100 mg
— Excipient q.s. pour une gélule terminée à 300 mg
(Détail de l'excipient : talc, stéarate de magnésium, aérosil).

Etude pharmacologique

a) Toxicité
La dose létale 50 ($DL_{50}$) a été évaluée après administration des produits par voie orale chez la souris.
Les résultats obtenus sont les suivants :
— Produit de l'exemple 1 $DL_{50}$ 350 mg/kg
— Produit de l'exemple 2 $DL_{50}$ 750 mg/kg

b) Détermination de l'activité antisécrétoire gastrique
La technique utilisée est décrite par H. Shay et al. dans Gastro enterology 5,43 (1945).

On utilise des rats mâles pesant environ 200 g privés de nourriture depuis 48 heures mais disposant à volonté de soluté glucosé à 8 %. On ligature le pylore des rats légèrement anesthésiés à l'éther, puis dès la fin de l'opération on administre le produit à tester à des doses diverses ou, pour les animaux témoins, une solution de carboxyméthyl cellulose à 0,5 % par voie intra-duodénale, puis on suture l'incision abdominale.

Trois heures plus tard, les animaux sont sacrifiés et leur estomac prélevé après ligature de l'œsophage. Le suc gastrique est prélevé et centrifugé. On relève alors le volume et sur 100 µl de suc gastrique, on détermine l'acidité totale par titrage à pH 7 à l'aide de soude N/100.

Les pourcentages de variation d'acidité totale des sécrétions gastriques sont calculés par rapport aux résultats obtenus avec les animaux témoins.

Les résultats sont les suivants : pour une dose de 10 mg/kg :
Produit de l'exemple 1 — 77 %
Produit de l'exemple 2 — 73 %

**Revendications** (pour les Etats contractants : BE, CH, DE, FR, GB, IT, LI, LU, NL, SE)

1. Les composés de formule (I) :

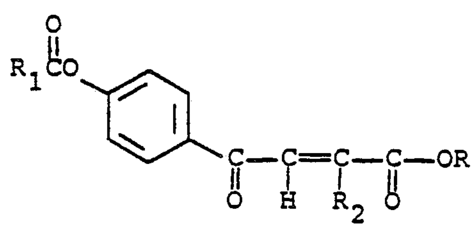

(I)

dans laquelle $R_1$ représente un radical alkyle renfermant de 1 à 18 atomes de carbone, $R_2$ représente un atome d'hydrogène ou un radical alkyle renfermant de 1 à 8 atomes de carbone et R représente un atome d'hydrogène ou un radical alkyle renfermant de 1 à 8 atomes de carbone, ainsi que les sels alcalins, alcalino-terreux ou d'amines des produits de formule (I) dans lesquels R représente un atome d'hydrogène.

2. Les composés de formule (I) tels que définis à la revendication 1, pour lesquels R représente un atome d'hydrogène, ainsi que leurs sels alcalins, alcalino-terreux ou d'amines.

3. Les composés de formule (I) tels que définis à l'une quelconque des revendications 1 ou 2, pour lesquels $R_2$ représente un atome d'hydrogène.

4. Les composés de formule (I) tels que définis à l'une quelconque des revendications 1 à 3, pour lesquels $R_1$ représente un radical méthyle ou un radical éthyle.

5. Les composés tels que définis à la revendication 1 dont les noms suivent :
— l'acide (E) 4-(4-acétoxyphényl) 4-oxo buten-2-oïque,
— l'acide (E) 4-(4-propanoyloxyphényl) 4-oxo buten 2-oïque,
ainsi que leurs sels alcalins, alcalino-terreux ou d'amines.

6. A titre de médicaments, les composés de formule (I) tels que définis à l'une quelconque des

revendications 1 à 4, ainsi que les sels alcalins, alcalino-terreux ou d'amines pharmaceutiquement acceptables des produits de formule (I) dans lesquels R représente un atome d'hydrogène.

7. A titre de médicaments, les composés de formule (I) tels que définis à la revendication 5, ainsi que leurs sels alcalins, alcalino-terreux ou d'amines pharmaceutiquement acceptables.

8. Les compositions pharmaceutiques renfermant comme principe actif au moins un médicament défini à l'une quelconque des revendications 6 ou 7.

9. Procédé de préparation des composés de formule (I) tels que définis à l'une quelconque des revendications 1 à 5, caractérisé en ce que l'on soumet un composé de formule (II) :

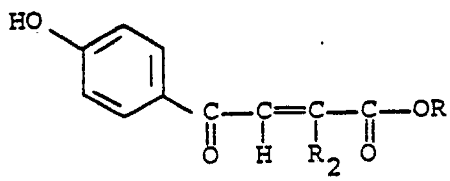

(II)

dans laquelle R et $R_2$ sont définis comme dans la revendication 1, à l'action d'un acide $R_1CO_2H$ ou d'un dérivé fonctionnel de cet acide, pour obtenir le composé de formule (I) correspondant que, le cas échéant, l'on salifie ou estérifie.

**Revendications** (pour l'Etat contractant AT)

1. Procédé pour préparer les composés de formule (I) :

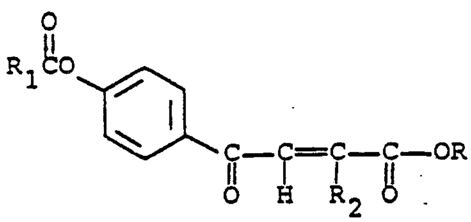

(I)

dans laquelle $R_1$ représente un radical alkyle renfermant de 1 à 18 atomes de carbone, $R_2$ représente un atome d'hydrogène ou un radical alkyle renfermant de 1 à 8 atomes de carbone et R représente un atome d'hydrogène ou un radical alkyle renfermant de 1 à 8 atomes de carbone, ainsi que les sels alcalins, alcalino-terreux ou d'amines des produits de formule (I) dans lesquels R représente un atome d'hydrogène, caractérisé en ce que l'on soumet un composé de formule (II) :

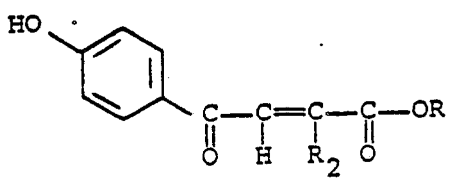

(II)

dans laquelle R et $R_2$ sont définis comme précédemment, à l'action d'un acide $R_1CO_2H$ ou d'un dérivé fonctionnel de cet acide, pour obtenir le composé de formule (I) correspondant que, le cas échéant, l'on salifie ou estérifie.

2. Procédé selon la revendication 1, caractérisé en ce que l'on utilise au départ, un composé de formule (II) dans laquelle R représente un atome d'hydrogène.

3. Procédé selon la revendication 1 ou 2, caractérisé en ce que l'on utilise au départ, un composé de formule (II) dans laquelle $R_2$ représente un atome d'hydrogène.

4. Procédé selon la revendication 1, 2 ou 3, caractérisé en ce que l'on utilise au départ un acide $R_1CO_2H$ ou un dérivé fonctionnel, dans lequel $R_1$ représente un radical méthyle ou éthyle.

**Claims** (for the Contracting States : BE, CH, DE, FR, GB, IT, LI, LU, NL, SE)

1. Compounds with the formula (I) :

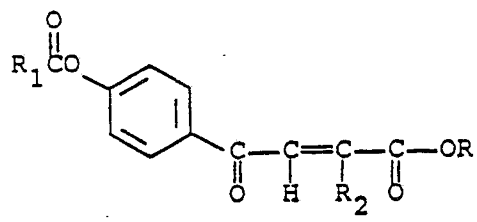

(I)

in which $R_1$ represents an alkyl radical containing from 1 to 18 carbon atoms, $R_2$ represents a hydrogen atom or an alkyl radical containing from 1 to 8 carbon atoms and R represents a hydrogen atom or an alkyl radical containing from 1 to 8 carbon atoms, as well as the alkali, alkaline-earth on amine salts of the products with the formula (I) in which R represents a hydrogen atom.

2. Compounds with the formula (I) as defined in claim 1, for which R represents a hydrogen atom, as well as their alkali, alkaline-earth or amine salts.

3. Compounds with the formula (I) as defined in any one of claims 1 or 2, for which $R_2$ represents a hydrogen atom.

4. Compounds with the formula (I) as defined in any one of claims 1 to 3, for which $R_1$ represents a methyl or ethyl radical.

5. Compounds as defined in claim 1 of which the names follow :
— (E) 4-(4-acetoxyphenyl)-4-oxobuten-2-oic acid,
— (E) 4-(4-propanoyloxyphenyl)-4-oxobuten-2-oic acid,
as well as their alkali, alkaline-earth or amine salts.

6. As medicaments, the compounds with the formula (I) as defined in any one of claims 1 to 4, as well as the pharmaceutically acceptable alkali, alkaline-earth or amine salts of the products with the formula (I) in which R represents a hydrogen atom.

7. As medicaments, the compounds with the formula (I) as defined in claim 5, as well as their pharmaceutically acceptable alkali, alkaline-earth or amine salts.

8. Pharmaceutical compositions containing as active principle at least one medicament defined in any one of claims 6 or 7.

9. Preparation process for compounds with the formula (I) as defined in any one of claims 1 to 5, characterised in that a compound with the formula (II) :

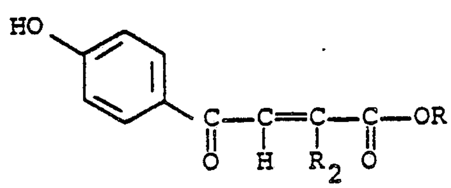

(II)

in which R and $R_2$ are defined as in claim 1, is submitted to the action of an acid $R_1CO_2H$ or a functional derivative of this acid, in order to obtain the corresponding compound with the formula (I) which, if necessary or desired, is salified or esterified.

**Claims** (for Contracting State AT)

1. Process for the preparation of compounds with the formula (I) :

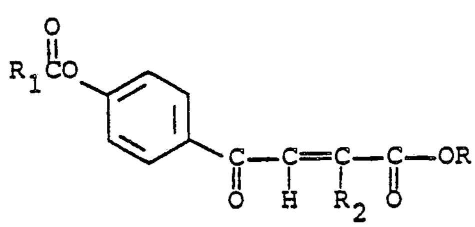

(I)

in which $R_1$ represents an alkyl radical containing from 1 to 18 carbon atoms, $R_2$ represents a hydrogen atom or an alkyl radical containing from 1 to 8 carbon atoms and R represents a hydrogen atom or an alkyl radical containing from 1 to 8 carbon atoms, as well as the alkali, alkaline-earth or amine salts of the products with the formula (I) in which R represents a hydrogen atom, characterized in that a compound with the formula (II) :

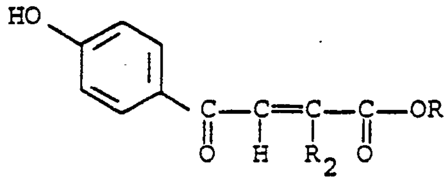

(II)

in which R and $R_2$ are defined as previously, is submitted to the action of an acid $R_1CO_2H$ or a functional derivative of this acid, in order to obtain the corresponding compound with the formula (I) which, if necessary or desired, is salified or esterified.

2. Process according to claim 1, characterised in that at the start a compound with the formula (II) is used, in which R represents a hydrogen atom.

3. Process according to claim 1 or 2, characterized in that at the start a compound with the formula (II) is used, in which $R_2$ represents a hydrogen atom.

4. Process according to claim 1, 2 or 3, characterized in that at the start an acid $R_1CO_2H$ is used, or a functional derivative of this acid, in which $R_1$ represents a methyl or ethyl radical.

**Patentansprüche** (für die Vertragsstaaten : BE, CH, DE, FR, GB, IT, LI, LU, NL, SE)

1. Verbindungen der Formel (I)

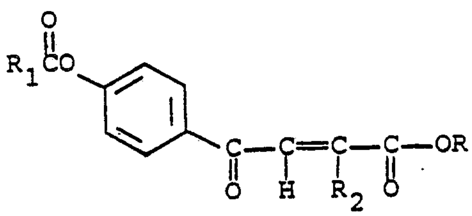

(I)

worin $R_1$ für einen Alkylrest mit 1 bis 18 Kohlenstoffatomen steht, $R_2$ ein Wasserstoffatom oder einen Alkylrest mit 1 bis 8 Kohlenstoffatomen bedeutet und R eine Wasserstoffatom oder einen Alkylrest mit 1 bis 8 Kohlenstoffatomen darstellt, sowie die Alkali-, Erdalkali- oder Aminsalze der Produkte der Formel (I), worin R für ein Wasserstoffatom steht.

2. Verbindungen der Formel (I) gemäß Anspruch 1, worin R ein Wasserstoffatom bedeutet, sowie deren Alkali-, Erdalkali- oder Aminsalze.

3. Verbindungen der Formel (I) gemäß einem der Ansprüche 1 oder 2, worin $R_2$ für ein Wasserstoffatom steht.

4. Verbindungen der Formel (I) gemäß einem der Ansprüche 1 bis 3, worin $R_1$ einen Methyl- oder Ethylrest darstellt.

5. Verbindungen gemäß Anspruch 1 mit den folgenden Bezeichnungen :
(E)-4-(4-Acetoxyphenyl)-4-oxo-buten-2-säure,
(E)-4-(4-Propanoyloxyphenyl)-4-oxo-buten-2-säure,
sowie deren Alkali-, Erdalkali- oder Aminsalze.

6. Als Arzneimittel die Verbindungen der Formel (I) gemäß einem der Ansprüche 1 bis 4 sowie die pharmazeutisch verträglichen Alkali-, Erdalkali- oder Aminsalze der Produkte der Formel (I), worin R für ein Wasserstoffatom steht.

7. Als Arzneimittel die Verbindungen der Formel (I) gemäß Anspruch 5 sowie deren pharmazeutisch verträgliche Alkali-, Erdalkali-, oder Aminsalze.

8. Pharmazeutische Zusammensetzungen, enthaltend als Wirkstoff zumindest ein Arzneimittel gemäß einem der Ansprüche 6 oder 7.

9. Verfahren zur Herstellung der Verbindungen der Formel (I) gemäß einem der Ansprüche 1 bis 5, dadurch gekennzeichnet, daß man eine Verbindung der Formel (II)

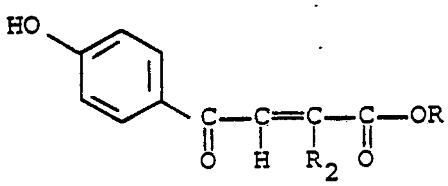

(II)

worin R und $R_2$ wie in Anspruch 1 definiert sind, der Einwirkung einer Säure $R_1CO_2H$ oder eines funktionellen Derivats dieser Säure unterzieht, um die entsprechende Verbindung der Formel (I) zu erhalten, die man gegebenenfalls in ein Salz überführt oder verestert.

**Patentansprüche** (für den Vertragsstaat AT)

1. Verfahren zur Herstellung der Verbindungen der Formel (I)

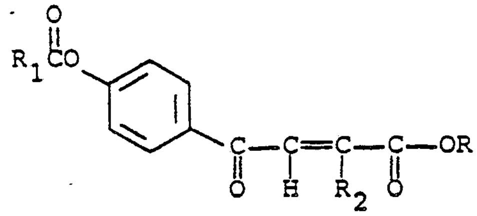

(I)

worin $R_1$ für einen Alkylrest mit 1 bis 18 Kohlenstoffatomen steht, $R_2$ ein Wasserstoffatom oder einen Alkylrest mit 1 bis 8 Kohlenstoffatomen bedeutet und R ein Wasserstoffatom oder einen Alkylrest mit 1 bis 8 Kohlenstoffatomen darstellt, sowie der Alkali-, Erdalkali- oder Aminsalze der Produkte der Formel (I), worin R für ein Wasserstoffatom steht, dadurch gekennzeichnet, daß man eine Verbindung der Formel (II)

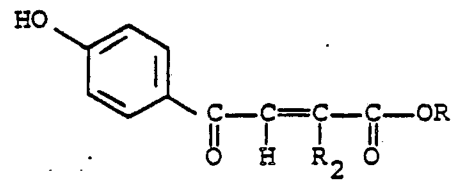

(II)

worin R und $R_2$ wie vorstehend definiert sind, der Einwirkung einer Säure $R_1CO_2H$ oder eines funktionellen Derivats dieser Säure unterzieht, um die entsprechende Verbindung der Formel (I) zu erhalten, die man gegebenenfalls in ein Salz überführt oder verestert.

2. Verfahren gemäß Anspruch 1, dadurch gekennzeichnet, daß man von einer Verbindung der Formel (II) ausgeht, worin R ein Wasserstoffatom bedeutet.

3. Verfahren gemäß Anspruch 1 oder 2, dadurch gekennzeichnet, daß man von einer Verbindung der Formel (II) ausgeht, worin $R_2$ ein Wasserstoffatom darstellt.

4. Verfahren gemäß Anspruch 1, 2 oder 3, dadurch gekennzeichnet, daß man von einer Säure $R_1CO_2H$ oder einem funktionellen Derivat, worin $R_1$ für einen Methyl- oder Ethylrest steht, ausgeht.